(19) Europäisches Patentamt / European Patent Office / Office européen des brevets

(11) **EP 4 115 895 A1**

(12) **EUROPEAN PATENT APPLICATION**
published in accordance with Art. 153(4) EPC

(43) Date of publication:
**11.01.2023 Bulletin 2023/02**

(21) Application number: **20922686.9**

(22) Date of filing: **02.12.2020**

(51) International Patent Classification (IPC):
**A61K 36/41** (2006.01)    **A61K 36/342** (2006.01)
**A61P 31/14** (2006.01)    **A23L 33/105** (2016.01)

(52) Cooperative Patent Classification (CPC):
(C-Sets available)
**A23F 3/00; A23L 33/105; A61K 36/342;**
**A61K 36/41; A61P 31/14**              (Cont.)

(86) International application number:
**PCT/KR2020/017454**

(87) International publication number:
**WO 2021/177553 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.03.2020 KR 20200027724**

(71) Applicant: **Nam, Jong Hyun**
**Seoul 05232 (KR)**

(72) Inventor: **Nam, Jong Hyun**
**Seoul 05232 (KR)**

(74) Representative: **Patentwerk B.V.**
**P.O. Box 1514**
**5200 BN 's-Hertogenbosch (NL)**

(54) **TEA COMPOSITION HAVING EFFICACY FOR PREVENTING OR IMPROVING RESPIRATORY DISEASES, AND PHARMACEUTICAL COMPOSITION COMPRISING SAME**

(57)    The present invention relates to an anti-respiratory virus composition, especially to a tea composition, and provides a natural composition having efficacy for treating and preventing respiratory virus-related diseases.

EP 4 115 895 A1

(52) Cooperative Patent Classification (CPC): (Cont.)

C-Sets
**A61K 36/342, A61K 2300/00;**
**A61K 36/41, A61K 2300/00**

**Description**

TECHNICAL FIELD

The present invention relates to an anti-respiratory virus composition, especially to a tea composition, and provides a natural composition having efficacy for treating and preventing respiratory virus-related diseases.

BACKGROUND ART

In general, the origin of viruses begins almost with the history of mankind, and has grown greatly to the present, causing tremendous suffering to mankind by its mutation. In particular, the virus propagated from civet cats caused tremendous suffering to mankind by showing massive toxicity called SARS, which was conquered with a plant extract called Tamiflu. MERS, which was bred from camels in the desert, terrorized mankind, but was also conquered with drugs extracted from plants such as Tamiflu. However, the situation is that MERS is still entering the endless latent period.

In particular, COVID-19, which is currently overwhelming the world including Wuhan, is a virus caused by a combination of a virus from a horseshoe bat to a wild mouse. After failing to develop vaccines and therapeutics through the study thereof in 2015, COVID-19 started appearing on Earth merged in 2020 and has been tremendously spreading. Human society has watched this tremendous spreading, and while struggling to develop vaccines or therapeutics, mankind is greatly embarrassed. In addition, there is no specific countermeasure except for primitive responses in very pre-modern methods such as hand-washing and masking. In the midst of inventing many kinds of foods, it was discovered that a chloroquine component could be detected from Isatidis Folium, and for the study, the chloroquine component was easily extracted and mixed with additional raw materials.

In particular, in the thought that mankind will not be able to solve the continuously occurring viruses forever, research into viruses seemed to have been loosened or neglected, but regrettably, many people are struggling with fear and diseases. In this regard, through continuous and earnest research, a finding that it is important to prevent and capture the final endpoint of a virus that is constantly mutated was attained, and it was also discovered that the resulting mutant virus was also killed, and natural tea was invented for prevention of viruses with secondary and tertiary mutations and for antiviral use.

DESCRIPTION OF EMBODIMENTS

TECHNICAL PROBLEM

An objective of the present invention is to provide a natural composition which is effective in the treatment and prevention of diseases caused by the appearance of respiratory viruses, particularly mutant viruses such as COVID-19.

Another objective of the present invention is to provide a pharmaceutical composition for an anti-respiratory virus without side effects.

Still another objective of the present invention is to provide a health functional food for an anti-respiratory virus without side effects.

SOLUTION TO PROBLEM

In order to achieve the objectives of the present invention, the present invention provides an anti-respiratory virus composition containing an Orostachys japonicus extract as an active ingredient.

According to an embodiment, when the respiratory virus is one or more selected from the group consisting of adeno virus, metapneumo virus, rhino virus, parainfluenza virus, influenza virus, respiratory syncytial virus, and coronavirus, the anti-respiratory virus composition is more effective.

According to an embodiment, the respiratory virus is preferably coronavirus.

According to an embodiment, the Isatidis tinctoria extract may be extracted by using one or more solvent selected from water and alcohols having 1 to 4 carbon atoms.

According to an embodiment, the anti-respiratory virus composition may further comprise an Adenophora triphylla extract.

According to an embodiment, the anti-respiratory virus composition may further comprise one or more selected from the group consisting of extracts of Codonopsis lanceolate, Asparagus cochinchinensis, Polygonatum odoratum, Astragali radix, Taraxacum playtcarpum, Scutellaria baicalensis Georgi, dry Zingiber officinal, Dioscorea batatas, Ginseng urealensis, Glycyrrhiza uralensis and Jujube, Codonopsis pilosulate radix, Atractylodes japonica, Dryopteris crassirhizoma, Ostericum koreanum, Agastache rugosa, Citrus unshiu peel, Schinzandra chinensis, Lycium chinense, Sophora japonica, Cuscuta semen, Salvia miltiorrhiza, Torilis japonica, Polygala tenuifolia and Curcuma longa.

The anti-respiratory virus composition is preferably provided as a health functional food and is preferably used in the form of a tea composition.

In order to achieve the objectives of the present invention, the present invention may comprise: an anti-respiratory virus composition according to the present invention; and a pharmaceutically acceptable carrier, excipient or diluent.

According to an embodiment, the pharmaceutical composition may have a formulation selected from the group consisting of tablets, pills, powders, granules, capsules, suspension emulsions, syrups, aerosols, external preparations, suppositories, and injections.

According to an embodiment, the pharmaceutical composition may be used for the treatment and prevention of anti-respiratory virus-related diseases.

According to an embodiment, the anti-respiratory virus composition according to the present invention and a foodologically acceptable supplementary additive may be included.

According to an embodiment, the health functional food may have a formulation selected from the group consisting of powders, granules, tablets, capsules, candies, chewing gums, jellies and beverages.

According to an embodiment, the health functional food may be used for the treatment and prevention of anti-respiratory virus-related diseases.

ADVANTAGEOUS EFFECTS OF DISCLOSURE

The anti-respiratory virus composition according to the present invention can be applied to various respiratory viruses, and in particular, can also be effective against mutant viruses caused by virus mutants. Thus, the anti-respiratory virus composition can quickly cope with viruses that appear as continuously mutated forms. In addition, when continuously used, immunity to various types of viruses is enhanced and thus a healthy state can be maintained.

The anti-respiratory virus composition according to the present invention can provide a pharmaceutical composition which is effective for the treatment or prevention of respiratory virus-related diseases, and is made of natural substances and thus there would be no side effects.

The anti-respiratory virus composition according to the present invention can provide a health functional food which is effective for the prevention of respiratory virus-related diseases, and can enhance immunity when continuously used.

BEST MODE

Hereinafter, the present invention will be described in more detail.

The present invention provides an anti-respiratory virus composition containing an Orostachys japonicus extract as an active ingredient.

When the respiratory virus is selected from the group consisting of adeno virus, metapneumo virus, rhino virus, parainfluenza virus, influenza virus, respiratory syncytial virus, and coronavirus, the anti-respiratory virus composition is more effective.

The respiratory virus may be a coronavirus, specifically a mutant virus, and more specifically a COVID 19 virus.

Coronavirus is a virus belonging to the family Coronaviridae, and very diverse diseases are caused by coronavirus infection. Coronavirus is an RNA virus that causes respiratory diseases, digestive diseases, liver diseases, and brain diseases in mammals and birds [Thomas M Gallagher TM, and Michael J Buchmeier MJ (2001) Coronavirus Spike Proteins in Viral Entry and Pathogenesis Virology, 279(2): 371-374]. Coronavirus is one of the viruses that cause the common cold (Baker SCPediatric Infect Dis J (2004) 23(11):1049-1050), but SARS virus is also a type of coronavirus. SARS virus first emerged in China in 2002, and by the spring of 2003, about 9,000 patients with severe acute respiratory disease in 32 countries around the world had a high mortality rate of about 15%. Additionally, coronavirus causes serious economic losses in the livestock industry as well. Another type of coronavirus, porcine epidemic diarrhea virus (PEDV), is a highly contagious viral disease that invades the gastrointestinal tract to cause dehydration due to vomiting and diarrhea and high fever and has high mortality, and thus causing significant economic losses [Duarte M, Laude H (1994) Sequence of the spike protein of the porcineepidemic diarrhoea virus J Gen Virol, 75 (Pt 5) : 1195-200]. In addition, swine-infectious transmissible gastroenteritis virus (TGEV), which has a high mortality in pigs, also belongs to coronavirus, which cause a variety of diseases in different animals, including mouse coronavirus causing hepatitis, rat coronavirus causing severe respiratory diseases, and bovine coronavirus causing neurological diseases [BrianDA, Baric RS Curr Top Microbiol Immunol (2005) 287: 1-30].

Orostachysjaponicus., also called Orosrachys malacophyllus, is a plant of the family Crassulaceae and inhabits while being attached to sunny rock crevices in the mountains or the roof tiles and stone walls of traditional houses. However, Orostachys japonicus is hard to find these days and is on the verge of extinction, so that, for medicinal use, the Orostachys japonicus is artificially cultivated and propagated. From old times, the Orostachys japonicus has been used to treat hemorrhoids, hepatitis, eczema, dysentery diarrhea, hemorrhoids, malignant boils, burns, etc., and it is known that rubbing same on boils or wounds is effective in absorbing pus. It is also known that the Orostachys japonicus is effective in promoting blood flow, pain relief, immunity, dermatitis, promoting hair growth, and dermatitis with cracked skin.

An extraction solvent that can be used for preparing the Orostachys japonicus extract may include, for example, water, a C1 to C4 lower alcohol, acetone, ethyl acetate, butyl acetate, and 1,3-butylene glycol, which can be used alone or in combination. In one embodiment, the extraction solvent is ethanol, more specifically about 70% ethanol. The amount of the extraction solvent is about 1 to 15 times the dry weight of Orostachys japonicus, and may be 5 to 12 times or 10 times, but not limited thereto. In addition, as the extraction method, hot water extraction, cold extraction, reflux cooling extraction, ultrasonic extraction, etc. may be used, and the extraction may be repeated once or multiple times. In addition, the extraction temperature is not particularly limited as long as the effective activity of the useful component of Orostachys japonicus is not removed. In one embodiment, the extraction is preferably performed by immersion at room temperature.

According to an embodiment of the present invention, an Adenophora triphylla extract may be further included.

The Adenophora tripphylla extract, which is an Adenophora triphylla leaf extract, may be extracted with, but not limited to, one solvent selected from the group consisting of water, lower alcohols having 1 to 4 carbon atoms, and mixtures thereof, and is preferably extracted with ethanol.

According to an embodiment of the present invention, one or more selected from the group consisting of extracts of Codonopsis lanceolate, Asparagus cochinchinensis, Polygonatum odoratum, Astragali radix, Taraxacum playtcarpum, Scutellaria baicalensis Georgi, dry Zingiber officinal, Dioscorea batatas, Ginseng urealensis, Glycyrrhiza uralensis and Zizyphus jujuba, Codonopsis pilosulate radix, Atractylodes japonica, Dryopteris crassirhizoma, Ostericum koreanum, Agastache rugosa, Citrus unshiu peel, Schinzandra chinensis, Lycium chinense, Sophora japonica, Cuscuta semen, Salvia miltiorrhiza, Torilis japonica, Polygala tenuifolia and Curcuma longa, may be further comprised.

The Codonopsis lanceolate extract may be included as an active ingredient to further enhance the efficacy of the anti-respiratory virus composition. The fermented Codonopsis lanceolate extract of the present invention can be prepared by various methods, which may include, for example, a method for obtaining an extract from a fermentation product of Codonopsis lanceolate by using a specific microorganism of the genus Bacillus, or a method for fermenting a Codonopsis lanceolate extract by using a specific microorganism of the genus Bacillus. Here, the specific microorganism of the genus Bacillus is selected from Bacillus subtilis, Bacillus coagulans, or a mixture thereof. In addition, in order to obtain the fermented Codonopsis lanceolate extract of the present invention, various organs or parts of Codonopsis lanceolate, for example, leaves, flowers, roots, stems, rhizomes, fruits, seeds, etc., can be used as raw materials, and, among these, roots of Codonopsis lanceolate is preferably used. Meanwhile, in order to obtain a fermented Codonopsis lanceolate extract from Codonopsis lanceolate, an extraction process is required, and as an extraction method, a general extraction method known in the art, for example, a solvent extraction method, may be used. The extraction solvent that can be used when preparing the fermented Codonopsis lanceolate extract by using the solvent extraction method may be selected from the group consisting of water, a C1 to C4 lower alcohol (e.g., methanol, ethanol, propanol and butanol) or a water-containing lower alcohol that is a mixture thereof, propylene glycol, 1,3-butylene glycol, glycerin, acetone, diethyl ether, ethyl acetate, butyl acetate, dichloromethane, chloroform, hexane and mixtures thereof, and among these, water, alcohol, or a mixture thereof is preferably selected. When water is used as the extraction solvent, the water is preferably hot water. In addition, when alcohol is used as the extraction solvent, the alcohol is preferably C1 to C4 lower alcohol, and the lower alcohol is more preferably selected from methanol and ethanol. In addition, when water-containing alcohol is used as the extraction solvent, the content thereof is preferably 50 to 90%.

By further systematically fractionating the Asparagus cochinchinensis extract extracted with water, alcohol or a mixture thereof in the order of n-hexane, methylene chloride, ethyl acetate and n-butanol, the present invention may comprise, as an active ingredient, methylene chloride, ethyl acetate, or ethyl acetate or n-butanol fraction fractionated with n-butanol as an active ingredient. As the Asparagus cochinchinensis roots, cultivated ones or commercially available ones may be used without limitation. In addition, the Asparagus cochinchinensis extract according to the present invention may be prepared in the following manner. The Asparagus cochinchinensis roots are thoroughly washed with water and then dried in the shade and at room temperature for 7 days. The dried Asparagus cochinchinensis roots are crushed and then placed in an extraction container to then be extracted for a certain period of time by using an extraction solvent at an appropriate temperature. After obtaining the Asparagus cochinchinensis extract, the solid content may be removed by using a filter paper or the like, the suspension may be centrifuged, and the supernatant may be filtered under reduced pressure. The extraction solvent may be water, alcohol or a mixture thereof, preferably a solvent selected from C1 to C4 lower alcohols or a mixed solvent thereof, and it is more preferable to use 70% ethanol, but not limited thereto. The amount of the extraction solvent is 2 to 10 times the dry weight of Asparagus cochinchinensis roots.

As the extraction method, an extraction method such as hot water extraction, immersion extraction, reflux cooling extraction, and ultrasonic extraction, may be used, and preferably, the extraction may be performed one to five times by using a hot water extraction method. During extraction, the extraction temperature is preferably 50°C to 100°C, and more preferably 50°C to 60°C. The extraction time is 1 to 4 hours, preferably 2 hours.

With regard to the Polygonatum odoratum extract, there are several types of Polygonatum odoratum, including Polygonatum inflatum, Polygonatum robustum,, Polygonatum involucratum, etc., and in Korea, there are 18 kinds of Polygonatum odoratum, including 16 species and 2 varieties (Lee Chang-Bok. 1995. Korean Plant Encyclopedia. Hyangmunsa Publishing House), both of which can be used for food, ornamental and medicinal purposes (Choi Young-Jeon. 1991, Method for cultivating and using wild vegetables. Ohseong Publishing House). Other names of Polygonatum odoratum may include Wi-su, Wi-yu, Polygonatum sibirium, Polygonatum officinale, Hwang-ji, Sopilkwanyeop, Seoninban, Tojook, Juknepul, Lonicera maackii, Polygonatum lasianthum var. coreanum, Polygonatum falcatum A. Gray, etc. In addition, the Polygonatum odoratum that can be used according to the method of the present invention may be any kind of Polygonatum odoratums, and can be used by collecting the roots thereof mainly in late autumn and early spring and washing same thoroughly.

As a method for producing the Polygonatum odoratum extract according to the present invention, a known method can be used. For example, Polygonatum odoratum roots, etc. are collected and are then cut into small pieces in the form of raw roots or after naturally drying same by sunlight or forcibly drying same in a mechanical manner, followed by adding a polar solvent such as water, methanol, ethanol, butanol, and a non-polar solvent such as ether, hexane, and chloroform, and performing immersion extraction at room temperature or warming extraction.

In a preferred embodiment of the present invention, the extract, which is obtained from a butanol layer prepared by heating Polygonatum odoratum at 80°C for 48 hours in a methanol solvent and then extracting, showed good physiological activities such as a hypoglycemic effect and an effect of lowering plasma lipid (total cholesterol and triglyceride, P<001). More specifically, the present inventors put dry Polygonatum odoratum and methanol in a grinder to crush same, and then the crushed Polygonatum odoratum-methanol solution was repeatedly extracted 1-3 times in an 80°C water bath for 48 hours by attaching a cooler and a mechanical stirrer, the extracted solution obtained by the above process was filtered by using a filter to obtain a methanol layer. Then, methanol contained in the methanol layer was removed by using a decompression device, and the methanol extract obtained by the above process was mixed in a ratio of methanol extract: $H_2O$: methanol: extraction solvent = 10: 9: 1: 10. Thereafter, extraction was sequentially performed by using hexane, chloroform, and n-butanol in that order as extraction solvents, thereby finally obtaining a butanol extract having a pharmacological effect.

The Astragali radix extract may be extracted by applying a method used for extraction of Astragali radix known in the art, and may be, for example, a solvent extraction method, but is not limited thereto. In addition, the Astragali radix extract may be prepared as a purified fraction by further performing an additional fractionation process using column chromatography. As the Astragali radix to be extracted, commercially available Astragali radix can be purchased and used, and it is preferable to use the purchased Astragali radix after removing foreign substances such as soil before being subjected to the extraction process.

The Taraxacum playtcarpum (Dandelion H DAHLST) is an outcrop of dandelion that is a perennial herb in the family Asteraceae, and contains taraxasterol, cholin, inulin, pectin, etc., specifically taraxol, taraxerol, Φ- taraxasterol, taraxasterol, β-amyrin, stigmasterol, β-sitosterol, cholin, organic acid, fructose, vitamin C, etc. in the roots thereof. As a pharmacological action of the Taraxacum playtcarpum, it is known that the Taraxacum playtcarpum is effective for gastritis and the like. The Taraxacum playtcarpum extract can be extracted by a method known in the art, and can also be obtained by the method disclosed in the present invention, for example, by using a water or organic solvent extraction method.

The Scutellaria baicalensis Georgi is a perennial plant belonging to the family Lamiaceae and is widely used as a medicinal plant in China, Japan, and Korea. With regard to the main efficacy, the Scutellaria baicalensis Georgi is known to be effective against inflammation, respiratory infections, and digestive tract infections. The Scutellaria baicalensis Georgi is known to have main components including baicalein, baicalin, wogonin, norwogonin, oroxylin A, β-sitosterol, mosloflavone, etc. The Scutellaria baicalensis Georgi extract of the present invention can be prepared as follows. After the dried outpost of the Scutellaria baicalensis Georgi was washed and minced, a solvent selected from water including purified water, a lower alcohol having 1 to 4 carbon atoms, such as methanol, ethanol, butanol, or a mixed solvent thereof, preferably water, methanol, ethanol, and a mixed solvent thereof, was mixed several times, and ultrasonic extraction, hot water extraction, room temperature extraction or reflux extraction, preferably room temperature extraction, was then repeatedly performed about 1 to 20 times, preferably 2 to 10 times, at 30°C to 150°C, preferably at room temperature, for 12 hours to 30 days, preferably 1 day to 7 days. As a result, the obtained extraction solution was filtered, concentrated under reduced pressure and dried, thereby obtaining a crude extract of the present invention.

As used herein, the term "Zingiber official" is a perennial grass native to tropical Asia belonging to the family Zingiber official of the genus Zingiber official. The family Zingiber official is mainly found in the tropics, and there are about 1,400 species in 47 genera around the world. About 50 species of Zingiber official are distributed in East Asia, India, and Malaysia. In Korea, Zingiber mioga and Zingiber official belonging to the genus Zingiber official are cultivated, flower Zingiber official in the genus Zingiber official flower is cultivated, and flower Zingiber mioga in the genus Zingiber mioga flower is growing in the forest soil of islands in the southern part of Korea. The Zingiber official has a height of about 40 to 100 cm, and the rhizomes thereof, which are the roots, are shaped of pulpy, slightly flattened and horizontally extending circles. The rhizomes of the Zingiber official are lumpy and branched, and are yellow in color and have a fragrant smell and a spicy and pungent taste. Leaves of the Zingiber officinal are alternate phyllotaxis, split into two, have no petiole, and long leaf sheaths surround the stem. The leaf body of the Zingiber officinal is linear lanceolate, 15-20 cm long, about 2cm wide, gradually pointed at the tip, and the base thereof is narrow, shiny, and hairless. Flowers of the Zingiber officinal are pale yellow, and ear inflorescence thereof are dense and about 7 cm long. Flowers of the Zingiber officinal bloom in July-September, but cultivated flowers thereof rarely bloom. The fruiting season of the Zingiber officinal is from December to January of the following year. The dried rhizome of the Zingiber officinal called dry Zingiber officinal, the rhizome cork bark thereof called Zingiber officinal husk, and the leaf thereof called Zingiber officinal leaf, are all used for medical purposes. In the present invention, the dry Zingiber officinal can be preferably used.

In the present invention, the Zingiber officinal extract can be obtained by: washing, drying, and pulverizing the Zingiber officinal; and extracting the pulverized Zingiber officinal with a solvent selected from water, C1-C4 lower alcohols, or mixed solvents thereof. The Zingiber officinal extract may comprise a product extracted by using a solvent selected from the group consisting of water, C1-C4 lower alcohols, and mixed solvents thereof, preferably methanol or ethanol, and more preferably methanol. In addition, in the present invention, the extract also comprises an extract obtained by extraction treatment, a diluted or concentrated solution of the extract, a dried product obtained by drying the extract, or any of these crude products or purified products.

The extraction method is not particularly limited, and the active ingredient may be extracted at room temperature or by heating under conditions in which the active ingredient is not destroyed or minimized. More specifically, the method for obtaining Zingiber officinal extract in the present invention is as follows. In extracting dried Zingiber officinal, as an extraction solvent, a polar solvent of water, a C1-C4 lower alcohol such as methanol, ethanol, butanol, etc. in a volume of about 2 to 20 times, preferably about 3 to 5 times the dry weight of the dried Zingiber officinal, or a mixed solvent thereof having a mixing ratio of 1:01 to about 1:10, was used. The extraction was performed at a temperature was 20 to 100, preferably 25 to 35 for about 5 hours to 10 days, preferably 5-48 hours, by using an extraction method such as shaking extraction, hot water extraction, cold extraction, reflux cooling extraction or ultrasonic extraction, etc. According to a specific embodiment of the present invention, after extracting the naturally-dried powder of Zingiber officinal with methanol at room temperature, the extract was filtered by using filter paper, and extracted by evaporation and concentration in a vacuum.

The Zingiber officinal extract may be extracted from various organs of natural, hybrid, and variegated plants, and may be extracted from, for example, roots, stems, leaves and flowers as well as plant tissue cultures. According to a specific embodiment of the present invention, the Zingiber officinal extract was prepared by recovering a mixture obtained by adding methanol to the dried root of Zingiber officinal and mixing, then concentrating and freeze-drying.

In the present invention, the fraction of the Zingiber officinal extract can be obtained by suspending the Zingiber officinal extract in MeOH and then fractionating same by using a polar solvent or a non-polar solvent to obtain a polar solvent fraction and a non-polar solvent fraction, respectively. As a specific embodiment in the present invention, as the solvent fraction, a MeOH fraction, a water fraction, an acetonitrile fraction, an n-hexane fraction, an ethyl acetate fraction, or a chloroform fraction may be provided. According to a specific embodiment of the present invention, the Zingiber officinal fraction was obtained by fractionation of the Zingiber officinal extract through silica gel column chromatography using CHCl3-MeOH elution. In addition, the solvent fraction may also be separated through column chromatography using, as a mobile phase, a mixed solvent consisting of two or more selected from a polar solvent such as $H_2O$, acetonitrile, methanol, ethanol, propanol or isopropanol and a non-polar solvent such as ethyl acetate, n-hexane, dichloromethane or chloroform, to then be used by isolating an active ingredient that is effective in the prevention and treatment of toxoplasmosis.

The Dioscorea batatas, which is a monocotyledonous perennial plant of the family Liliaceae, is also called Sanwoo or Seoyeo, is native to China and cultivated as a medicinal herb, and grows wild in mountainous areas. The tuber root of Dioscorea batatas contains 15 to 20% of starch as a main ingredient and various ingredients including various amino acids such as arginine, histidine, lysine, and tryptophan, as well as minerals such as calcium, sodium, magnesium and potassium, as well as vitamin B1 and vitamin C. In the present invention, tuber roots of Dioscorea batatas are used.

The Glycyrrhiza uralensis is a perennial plant belonging to the family Legume, and is native to or cultivated in northern China, Siberia, southern Italy, Manchuria, and Mongolia. The Glycyrrhiza uralensis is one of medicinal herbs used as an antitussive while being included in all herbal medicine prescriptions as an ingredient to reduce toxicity (Rauchensteiner F et al, JPharmBiomedAnal, 38(4), pp. 594-600, 2005), and in Europe, the Glycyrrhiza uralensis may also be used to sweeten tobacco, gum, and candy According to the results of a recent study, the Glycyrrhiza uralensis relieves oxidative damage to the kidney (YOKOZAWA T. et al, FreeRadic Res, 39(2), pp. 203-211, 2005), and it has been confirmed that the Glycyrrhiza uralensis also has an effect of protecting hepatocytes damaged by cadmium (KIM S.C. et al, Toxicology, 197(3), pp. 239-251, 2004).

In the present invention, Glycyrrhiza uralensis may be purchased and used commercially, or directly harvested or grown in nature. In the present invention, commercially available Glycyrrhiza uralensis may be purchased and used, or Glycyrrhiza uralensis harvested or grown directly from nature may be used. In a method for preparing the extract of Glycyrrhiza uralensis, a conventional extraction method used in the art, such as ultrasonic extraction, filtration, reflux extraction, etc., may be used. Preferably, the Glycyrrhiza uralensis may be an extract obtained by pulverizing Glycyrrhiza uralensis from which foreign substances have been removed by washing and drying, and extracting the dried product of Glycyrrhiza uralensis with water, C1-C4 alcohol, or a mixed solvent thereof, more preferably an extract extracted with C1-C4 alcohol, and most preferably an extract extracted with methanol or ethanol. Here, the extraction solvent is preferably 2 to 20 times the dry weight of Glycyrrhiza uralensis. For example, after shredding, the dried product of Glycyrrhiza uralensis may be put into an extraction container, and C1-C4 lower alcohols or a mixed solvent thereof, preferably methanol or ethanol, may be added thereto and left at room temperature for a certain period of time, followed by filtering, thereby obtaining an alcohol extract. Here, the extraction is preferably left at room temperature for one week, and a method, such as concentration or freeze-drying, may then be additionally performed.

Zizyphus jujuba, which is a health food since ancient times, is rich in nutrients, and is an essential ingredient in the oriental medicine together with Glycyrrhiza uralensis. It has been reported that Zizyphus jujuba basically contains a lot of carbohydrates and ascorbic acid and includes, as medicinal ingredients, various kinds of sterols, alkaloids, saponins, vitamins, organic acids, amino acids, etc., and pharmacological effects thereof, such as laxative, diuretic, tonic, cholestasis, stimulant, stamina recovery, expectorant, and anti-inflammatory effects, have also been reported and identified, so that Zizyphus jujuba is widely used in the pharmaceutical industry. Zizyphus jujubas, which have recently been spotlighted as alternative crops of apples and grapes, the market of which has been eroding due to the import opening of agricultural products, are being promoted as high-income fruit trees, and the cultivation area and yield thereof are gradually increasing.

Extracts of Codonopsis pilosulate, Atractylodes japonica, Dryopteris crassirhizoma, Ostericum koreanum, Agastache rugosa, Citrus unshiu peel, Schinzandra chinensis, Lycium chinense, Sophora japonica, Cuscuta semen, Salvia miltiorrhiza, Torilis japonica, Polygala tenuifolia, and Curcuma longa, which are added according to an embodiment of the present invention, may be extracted by a method known to a person skilled in the art.

The composition of the present invention may further comprise honey.

The anti-respiratory virus composition according to the present invention preferably contains 10 to 30% by weight of Orostachys japonicus extract on the basis of the total composition.

The anti-respiratory virus composition according to the present invention is preferably included in an amount of 5 to 15% by weight on the basis of the total composition.

The anti-respiratory virus composition according to the present invention preferably contains Orostachys japonicus extract and Adenophora triphylla extract in a ratio of 2-6: 1-3 parts by weight.

The anti-respiratory virus composition according to an embodiment of the present invention preferably contains at least one selected from the group consisting of one or more selected from the group consisting of extracts of Codonopsis lanceolate, Asparagus cochinchinensis, Polygonatum odoratum, Astragali radix, Taraxacum playtcarpum, Scutellaria baicalensis Georgi, dry Zingiber officinal, Dioscorea batatas, Ginseng urealensis, Glycyrrhiza uralensis and Zizyphus jujuba, Codonopsis pilosulate radix, Atractylodes japonica, Dryopteris crassirhizoma, Ostericum koreanum, Agastache rugosa, Citrus unshiu peel, Schinzandra chinensis, Lycium chinense, Sophora japonica, Cuscuta semen, Salvia miltiorrhiza, Torilis japonica, Polygala tenuifolia and Curcuma longa, in an amount of 50 to 70% by weight on the basis of the total weight of the composition.

According to another aspect of the present invention, a pharmaceutical composition for anti-respiratory virus may be provided by further adding a pharmaceutically acceptable carrier, excipient or diluent to the anti-respiratory virus composition according to the present invention.

The present invention provides health functional food comprising the anti-respiratory virus food composition. Health functional food refers to food that is added to food materials such as beverages and teas, or manufactured by encapsulation, powdering, suspension, etc., which means that, when consumed, the health functional food brings about a specific effect on health. However, unlike general drugs, the health functional food is advantageous in that there are no side effects, which may occur when drugs are taken for a long time because food is used as a raw material. The thus obtained health functional food of the present invention can be ingested on a daily basis and thus is very useful. In such health food, the amount of the anti-respiratory virus composition added may vary depending on the type of target health food and cannot be uniformly specified. However, the amount of the anti-respiratory virus composition added is generally in the range of 001 to 50% by weight, preferably 01 to 20% by weight, on the basis of the target food. In addition, in the case of food in the form of granules, tablets or capsules, the anti-respiratory virus composition is generally added in an amount of 01 to 100% by weight, preferably 05 to 80% by weight. In one embodiment, the health functional food of the present invention may be in the form of a beverage.

The food composition or health functional food according to the present invention can be usefully used as a supplement to be taken before or after administration of an anti-respiratory virus therapeutic agent.

MODE OF DISCLOSURE

Advantages and features of the present invention, and methods of achieving same, will become apparent with reference to the embodiments described below in detail. However, the present invention is not limited to the embodiments disclosed below but will be embodied in various different forms, and only these embodiments allow the disclosure of the present invention to be complete and are provided so that this disclosure will be thorough and complete and will convey the aspects and features of the present disclosure to those skilled in the art. The present invention is merely defined by the scope of the appended claims.

**Preparation Example 1: Preparation of Orostachys japonicus extract**

To prepare the Orostachys japonicus extract, 20-fold distilled water was added to Orostachys japonicus on the basis of the weight of Orostachys japonicus, hot-water extraction was performed at 115 °C for 180 minutes, and the precipitate was removed by primary filtration at 0.45 μm, followed by secondary filtration at 0.22 μm. Next, the pH was adjusted to 7.0, and each 1 ml of the filtered product was dispensed into a 1.5 ml Ep-tube and stored at -20°C for use in all analyses.

**Preparation Example 2: Preparation of Adenophora triphylla extract**

The leaves, stems and roots of Adenophora triphylla were air-dried and pulverized, then 10-fold methanol was added and extracted twice for each 12 hours. The filtrate was collected, the solvent was evaporated in a rotary evaporator, and the remaining residue was partially taken and frozen (at -70°C) while the remainder was used for sequential solvent fractionation. That is, after the solvent fractionation was sequentially performed with hexane, ethyl acetate, and distilled water (water), the solvent was evaporated in a rotary evaporator and dried to be used as a sample.

**Preparation Example 3: Preparation of extracts of Codonopsis lanceolate, Asparagus cochinchinensis, Polygonatum odoratum, Astragali radix, Taraxacum playtcarpum, Scutellaria baicalensis Georgi, Dry Zingiber officinal, Dioscorea batatas, Ginseng urealensis, Glycyrrhiza uralensis, Zizyphus jujuba, Codonopsis pilosulate, Atractylodes japonica, Dryopteris crassirhizoma, Ostericum koreanum, Agastache rugosa, Citrus unshiu peel, Schinzandra chinensis, Lycium chinense, Sophora japonica, Cuscuta semen, Salvia miltiorrhiza, Torilis japonica, Polygala tenuifolia and Curcuma longa**

The respective plants were washed and weighed, and the extracts were prepared according to the method of Preparation Example 1.

**Example 1:**

The Orostachys japonicus extract prepared in Preparation Example 1 was used.

**Example 2:**

The extract of Preparation Example 1 and the extract of Preparation Example 2 were mixed in a weight ratio of 10: 5, and the mixed extract was used.

**Examples 3 to 5:**

The compositions were prepared by using the extracts of Preparation Examples 1 to 3 having the composition shown in Table 1 below.

[Table 1]

| No. | Raw material | Main compounding ratio (wt%) | | |
|---|---|---|---|---|
| | | Example 3 | Example 4 | Example 5 |
| 1 | Orostachys japonicus | 15 | 25 | 10 |
| 2 | Adenophora triphylla | 7 | 5 | 10 |
| 3 | Codonopsis lanceolate | 5 | 3 | 5 |

(continued)

| No. | Raw material | Main compounding ratio (wt%) | | |
|---|---|---|---|---|
| | | Example 3 | Example 4 | Example 5 |
| 4 | Asparagus cochinchinens | 5 | 3 | 3 |
| 5 | Polygonatum odoratum | 10 | 8 | 2 |
| 6 | Astragali radix | 10 | 8 | 3 |
| 7 | Taraxacum playtcarpum | 5 | 7 | 3 |
| 8 | Scutellaria baicalensis Georgi | 5 | 7 | 2 |
| 9 | Dry Zingiber officinal | 5 | 5 | 4 |
| 10 | Dioscorea batatas | 5 | 4 | 5 |
| 11 | Ginseng urealensis | 5 | 3 | 3 |
| 12 | Glycyrrhiza uralensis | 3 | 2 | 1 |
| 13 | Zizyphus jujuba | 10 | 10 | 5 |
| 14 | Honey | 10 | 10 | 10 |
| 15 | Codonopsis pilosulate | - | - | 5 |
| 16 | Atractylodes japonica | - | - | 2 |
| 17 | Dryopteris crassirhizoma | - | - | 1 |
| 18 | Ostericum koreanum | - | - | 3 |
| 19 | Agastache rugosa | - | - | 4 |
| 20 | Citrus unshiu peel | - | - | 4 |
| 21 | Schinzandra chinensis | - | - | 2 |
| 22 | Lycium chinense | - | - | 1 |
| 23 | Sophora japonica | - | - | 3 |
| 24 | Cuscuta semen | - | - | 3 |
| 25 | Salvia miltiorrhiza | - | - | 3 |
| 26 | Torilis japonica | - | - | 1 |
| 27 | Polygala tenuifolia | - | - | 1 |
| 28 | Curcuma longa | - | - | 1 |
| Total | | 100 | 100 | |

**Experimental Example 1: Virus Culture**

The target viruses used to measure the antiviral activity of the aqueous extracts obtained in Examples 1 to 5 were infectious gastroenteritis virus (TGEV) and porcine epidemic diarrhea virus (PEDV), and TGEV was cultured in ST cells, and PEDV was cultured in Vero cells. The ST cells and the Vero cells were cultured in a 37 °C incubator using a minimal essential medium (MEM) containing 10% fetal serum.

**Experimental Example 2: Analysis of Virus Inhibitory Ability**

To measure the virus proliferation inhibitory ability of the aqueous extracts obtained in Examples 1 to 5 against TGEV, ST cells and Vero cells were cultured in 96-well plates and each cell was at least 90% filled at the bottom of the well. The existing culture medium was removed, and a new culture medium containing TGEV was administered to each well, and each plant extract was administered to each well by concentration (250, 500, 1000 µg/ml media). The virus proliferation inhibitory ability of each extract was measured by TGEV-infecting ST or Vero cells for 48 hours, followed by measuring live cells through SRB assay [Martin A, Martin C (1997) Comparison of 5 microplate colorimetric assay for in vitro cytoxicity testing and cell proliferation assay Cytotechnology, 11: 49-54]. 100 μl of 10% trichloroacetic acid (TCA) was added to each well, and the resultant product was left at 4 °C for 1 hour and washed several times with distilled water. After drying at room temperature, 100 μl of a 04% (w/v) SRB (sulforhodamine B) solution dissolved in 1% (v/v) acetic acid was added and stained for 30 minutes. The SRB staining solution that was not bound to cells was washed several times with 1% (v/v) acetic acid and then dried again. The morphology of the cells at the bottom of each well was photographed by using a microscope camera (Zeiss, Model Axivert10), and 100 μl of a 10 mM Tris solution (pH 105) was added to each well to sufficiently dissolve the cell-bound dye. Then, absorbance was measured at 560 nm. Treatment groups, including a group that is not treated with virus, a group that is treated only with plant extracts (Examples 1 to 3), a group that is treated with only virus, and a group that is treated with virus and water-soluble extracts, were marked with A, B, C and D, respectively, and the virus proliferation inhibitory ability (%) [Equation 1] of each plant extract was calculated.

[Equation 1]

Virus inhibitory ability (%)=[(D-C)/(B-C)]x100

**Experimental Result 1: Analysis of Antiviral Activity against TGEV**

The antiviral activity against TGEV of all of the extracts of Examples 1 to 5 are shown in Table 2 below. Examples 1 to 5 showed relatively high virus proliferation inhibitory ability, and, specifically, the extract of Example 5 was the highest.

[Table 2]

|  | Antiviral activity against TGEV (%) | | | |
| --- | --- | --- | --- | --- |
|  | Uninfected cells | 250 μg/ml | 600 μg/ml | 1000 μg/ml |
| Example 1 | 100 ±5.55 | 179.55 ±20.55 | 167.55 ±25.36 | 180.25 ±66.33 |
| Example 2 | 100 ±8.00 | 180.42 ±22.36 | 182.22 ±11.93 | 190.66 ±87.52 |
| Example 3 | 100 ±9.96 | 195.85±50.55 | 180.33 ±180.70 | 198.40 ±86.55 |
| Example 4 | 100 ±8.96 | 197.85±55.55 | 185.43 ±180.22 | 199.10 ±87.54 |
| Example 5 | 100 ±9.90 | 195.85±50.55 | 180.33 ±180.70.22 | 200.40 ±70.55 |

**Claims**

1. An anti-respiratory virus composition containing an Orostachysjaponicus extract as an active ingredient.

2. The anti-respiratory virus composition of claim 1, wherein the respiratory virus is one or more selected from the group consisting of adeno virus, metapneumo virus, rhino virus, parainfluenza virus, influenza virus, respiratory syncytial virus, and coronavirus.

3. The anti-respiratory virus composition of claim 1, wherein the respiratory virus is coronavirus.

4. The anti-respiratory virus composition of claim 1, wherein the Isatidis tinctoria extract is extracted by using one or more solvent selected from water and alcohols having 1 to 4 carbon atoms.

5. The anti-respiratory virus composition of claim 1, further comprising an Adenophora triphylla extract.

6. The anti-respiratory virus composition of claim 1, further comprising one or more selected from the group consisting of extracts of Codonopsis lanceolate, Asparagus cochinchinensis, Polygonatum odoratum, Astragali radix, Taraxacum playtcarpum, Scutellaria baicalensis Georgi, dry Zingiber officinal, Dioscorea batatas, Ginseng urealensis, Glycyrrhiza uralensis and Zizyphus jujuba, Codonopsis pilosulate radix, Atractylodes japonica, Dryopteris crassirhizoma, Ostericum koreanum, Agastache rugosa, Citrus unshiu peel, Schinzandra chinensis, Lycium chinense, Sophora japonica, Cuscuta semen, Salvia miltiorrhiza, Torilis japonica, Polygala tenuifolia and Curcuma longa.

7. A pharmaceutical composition for an anti-respiratory virus, comprising: the anti-respiratory virus composition according to any one of claims 1 to 6; and a pharmaceutically acceptable carrier, excipient or diluent.

8. The pharmaceutical composition of claim 7, wherein the pharmaceutical composition has a formulation selected from the group consisting of tablets, pills, powders, granules, capsules, suspension emulsions, syrups, aerosols, external preparations, suppositories, and injections.

9. The pharmaceutical composition of claim 7, being effectively used in the treatment and prevention of anti-respiratory virus-related diseases.

10. A health functional food for an anti-respiratory virus, comprising: the anti-respiratory virus composition according to any one of claims 1 to 6; and a foodologically acceptable supplementary additive.

11. The health functional food of claim 10, wherein the health functional food has a formulation selected from the group consisting of powders, granules, tablets, capsules, candies, chewing gums, jellies and beverages.

12. The health functional food of claim 10, wherein the health functional food is a health functional tea.

13. The health functional food of claim 10, wherein the health functional food is effectively used in the prevention of respiratory virus-related diseases.

## INTERNATIONAL SEARCH REPORT

| International application No. |
|---|
| **PCT/KR2020/017454** |

**A. CLASSIFICATION OF SUBJECT MATTER**

**A61K 36/41**(2006.01)i; **A61K 36/342**(2006.01)i; **A61P 31/14**(2006.01)i; **A23L 33/105**(2016.01)i

According to International Patent Classification (IPC) or to both national classification and IPC

**B. FIELDS SEARCHED**

Minimum documentation searched (classification system followed by classification symbols)

A61K 36/41(2006.01); A23L 1/30(2006.01); A23L 33/00(2016.01); A23L 33/105(2016.01); A61K 36/15(2006.01); A61K 36/8965(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 바위솔(Orostachys japonicus), 추출물(extract), 호흡기 질환(respiratory disease), 차(tea)

**C. DOCUMENTS CONSIDERED TO BE RELEVANT**

| Category* | Citation of document, with indication, where appropriate, of the relevant passages | Relevant to claim No. |
|---|---|---|
| X<br>Y | PARK, J. G. et al. Phenolic Compounds from Orostachys japonicus having Anti-HIV-1 Protease Activity. Natural Product Sciences. 2000, vol. 6, no. 3, pp. 117-121.<br>    See abstract. | 1-4,7-13<br>5,6 |
| Y | 강원도 잔대(사삼) 효능, 효과/ 호흡기 질환에 좋은 약초. 네이버 블로그. 06 September 2017. non-official translation (Gangwon-do Zandae (Sasam) Efficacy and Effect/ A Medicinal Herb that is Good for Respiratory Diseases. Naver blog). [URL: https://blog.naver.com/PostPrint.nhn?blogId=frelee2&logNo=221090664766].<br>    See page 1. | 5 |
| Y | KR 10-2017-0003153 A (LEE, Chung Ryul) 09 January 2017 (2017-01-09)<br>    See abstract; and claim 1. | 6 |
| A | KR 10-2018-0040912 A (JEJU TECHNOPARK) 23 April 2018 (2018-04-23)<br>    See entire document. | 1-13 |

☑ Further documents are listed in the continuation of Box C. ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 March 2021** | **22 March 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| | International application No. |
|---|---|
| | **PCT/KR2020/017454** |

### C.    DOCUMENTS CONSIDERED TO BE RELEVANT

| Category* | Citation of document, with indication, where appropriate, of the relevant passages | Relevant to claim No. |
|---|---|---|
| A | KR 10-2014-0072464 A (INJE UNIVERSITY INDUSTRY-ACADEMIC COOPERATION FOUNDATION) 13 June 2014 (2014-06-13)<br>See entire document. | 1-13 |
| A | KR 10-1943475 B1 (KANG, Soo Young) 29 January 2019 (2019-01-29)<br>See entire document. | 1-13 |

## INTERNATIONAL SEARCH REPORT
### Information on patent family members

International application No.

**PCT/KR2020/017454**

| Patent document cited in search report | | | Publication date (day/month/year) | Patent family member(s) | | | Publication date (day/month/year) |
|---|---|---|---|---|---|---|---|
| KR | 10-2017-0003153 | A | 09 January 2017 | None | | | |
| KR | 10-2018-0040912 | A | 23 April 2018 | KR | 10-2018-0128365 | A | 03 December 2018 |
| KR | 10-2014-0072464 | A | 13 June 2014 | None | | | |
| KR | 10-1943475 | B1 | 29 January 2019 | None | | | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **THOMAS M ; GALLAGHER TM ; MICHAEL J ; BUCHMEIER MJ.** *Coronavirus Spike Proteins in Viral Entry and Pathogenesis Virology,* 2001, vol. 279 (2), 371-374
- **BAKER SC.** *Pediatric Infect Dis J,* 2004, vol. 23 (11), 1049-1050
- **DUARTE M ; LAUDE H.** Sequence of the spike protein of the porcineepidemic diarrhoea virus. *J Gen Virol,* 1994, vol. 75, 1195-200
- **BRIANDA ; BARIC RS.** *Curr Top Microbiol Immunol,* 2005, vol. 287, 1-30
- **LEE CHANG-BOK.** Korean Plant Encyclopedia. Hyangmunsa Publishing House, 1995
- **CHOI YOUNG-JEON.** Method for cultivating and using wild vegetables. Ohseong Publishing House, 1991
- **RAUCHENSTEINER F et al.** *JPharmBiomedAnal,* 2005, vol. 38 (4), 594-600
- **YOKOZAWA T. et al.** *FreeRadic Res,* 2005, vol. 39 (2), 203-211
- **KIM S.C. et al.** *Toxicology,* 2004, vol. 197 (3), 239-251
- **MARTIN A ; MARTIN C.** Comparison of 5 microplate colorimetric assay for in vitro cytoxicity testing and cell proliferation assay. *Cytotechnology,* 1997, vol. 11, 49-54